# EUROPEAN PATENT SPECIFICATION

(11) **EP 3 160 323 B1**
(45) Date of publication and mention of the grant of the patent: **07.04.2021**
(21) Application number: 14896125.3
(22) Date of filing: 25.06.2014
(51) Int. Cl.: A61M 25/01, A61M 25/02, A61B 1/00, A61B 5/00

(54) **ULTRATHIN ENDOSCOPE AUXILIARY SYSTEM AND METHOD OF USE**
ULTRADÜNNES ENDOSKOPHILFSSYSTEM UND VERFAHREN ZUR VERWENDUNG
SYSTÈME AUXILIAIRE D'ENDOSCOPE ULTRA-MINCE ET PROCÉDÉ D'UTILISATION

(43) Date of publication of application: 03.05.2017
(73) Proprietor: MacKay Memorial Hospital, Taipei City 104 (TW)
(72) Inventor: Liu, Chia-Yuan, Taipei City, 122 (TW); Lin, Ching-Chung, Taipei City, 111 (TW)
(74) Representative: Lang, Christian
(86) International application number: PCT/CN2014/080688
(87) International publication number: WO 2015/196380

(56) References cited:
- WO-A1-2008/026445
- WO-A1-2010/089923
- CN-A- 101 238 969
- CN-A- 103 584 827
- CN-Y- 200 939 121
- JP-A- 2008 035 908
- JP-A- 2008 035 909
- JP-A- 2012 205 717
- US-A1- 2011 230 716

## Description

### BACKGROUND OF THE INVENTION

### 1 . FIELD OF THE INVENTION

The present disclosure in general relates to the field of endoscope; and more particularly to an ultrathin endoscope auxiliary system for use with an ultrathin endoscope.

### 2. DESCRIPTION OF RELATED ART

The occurrence of biliary diseases have been rising in recent year, thus results in a pressing need in clinical practice for an improved instrument and/or technique that may provide direct access to the bile duct, so that better diagnosis and/or treatment of biliary diseases may be rendered thereto. Currently, endoscopic retrograde cholangio-pancreatography (ERCP) is the main tool to diagnose and/or treat the biliary diseases. In conventional ERCP, a contrast agent is injected into the bile duct and pancreatic duct of a patient, and the lesion, if any, is then observed with the aid of X-rays. However, unlike other endoscopic techniques, the ERCP does not allow the physician a direct view of the lesion, and hence, during the ERCP procedure, attending physician cannot blow up the lesion image, nor performs staining or biopsy at the lesion. As to the treatment of bile duct disease, nor can the ERCP procedure allow an endoscope to be inserted into the bile duct, thereby limiting the application of ERCP in many endoscopic treatments, such as electrohydraulic lithotripsy, hemostasis and tumor ablation.

The later developed direct peroral cholangioscopy (DPOCS) addresses the aforementioned disadvantages of the ERCP. The DPOCS allows a physician to image and/or treat the lesion directly; thus, DPOCS is highly appraised by the skill artisans in the art.

The endoscopic system suitable for use in the DPOCS can be a single endoscope system (i.e., single light source/single direction) or dual endoscope system (i.e., dual light source/bi-direction).

The mother-and-baby-scope is the most commonly used dual endoscope system in the clinical setting. In practice, the operation of the mother-and-baby-scope requires two physicians working side-by-side simultaneously, which renders the labor cost for its operation extremely high. Moreover, the mother-and-baby-scope consists of two individual endoscopes respectively coupled with two individual light sources. However, operation of the baby scope often fails, which leads to a higher maintenance fee of the mother-and-baby-scope. Accordingly, the SpyGlassTM system (Boston Scientific) was developed as an alternative to the conventional mother-and-baby-scope system. However, the resolution of the SpyGlassTM system is poor, nor can it perform Narrow Band Imaging (NBI) and/or Image Enhanced Endoscopy (IEE) that may improve the proficiency of diagnosis. Moreover, the SpyGlassTM system is equipped with narrow instrument conduits, thereby limits the selection of medical instrument suitable for inserting into the SpyGlassTM system. In view of the foregoing, it appears that the SpyGlassTM system has limited applications in the clinical setting, not to mention the equipment and accessories of the SpyGlassTM system are expensive, thereby rendering the SpyGlassTM system the last choice in clinical practice.

As to the single endoscope system, the ultrathin endoscope is the main tool selected to carry out the DPOCS, however, the operation is technique-intensive. Further, the ultrathin endoscope system has its own limits. For example, while passing the ultrathin endoscope through the mouth, esophagus, stomach and reaching Ampulla (Papilla) of Vater of the second portion of the duodenum connected to the opening of the bile duct, the front end of the ultrathin endoscope needs to be bent by 180 degrees, so that it can be disposed in front of the opening of the Ampulla of Vater. However, when the user (i.e., the attending physician) tries to push the ultrathin endoscope forward and into the bile duct, the pushing force is likely to cause the ultrathin endoscope falls out of the bile duct, due to the fact that ultrathin endoscope is reversed by 180 degrees. By contrast, if the user pulls back the endoscope, the pulling force is likely to cause the ultrathin endoscope being stuck in the Ampulla of Vater instead of proceeding forward into the bile duct. Furthermore, even though the front end of the ultrathin endoscope is bent by more than 180 degrees, it will still not enough to allow itself being disposed in front of the Ampulla of Vater. Therefore, implementing DPOCS by use of ultrathin endoscope remains troublesome in the clinical field.

Last, but not least, the long and thin structure of the ultrathin endoscope tends to bend or loop in the stomach of the patient. When that happens, it is difficult to properly adjust the axial orientation and/or the length of the ultrathin endoscope in the gastrointestinal tract of the patient, causing the ultrathin endoscope fails to reach the Ampulla of Vater, and the inevitable failure of DPOCS.

To attack the inherent looping problem of the ultrathin endoscope, Takao Itoi et al. proposed an improved multi-bending ultrathin endoscope (Digestive Endosccopy; 2013, doi; 7.10.1111/den.12082). Unfortunately, clinical surgical success rate of this improved ultrathin endoscope wasn't high enough (7/41, 17%). Waxman *et al.* then proposed using a balloon as a positioning member disposed on the outer wall of the ultrathin endoscope to assist the performance of DPOCS. However, due to safety concern, this improved ultrathin endoscope of Waxman et al. has been withdrawn from the market (GASTROINTESTINAL ENDOSCOPY, 2010, 72(5), p1052-1056.).

Document JP 2012 205717 A discloses a conventional endoscope auxiliary system, wherein such endoscope auxiliary system comprises an overtube (64) configured to receive the insertion portion (12) of an endoscope therein, wherein the overtube is a tubular body comprising a tip opening portion (67) at one end thereof and an insertion passage (66) inside the overtube. On the side wall (65) of the overtube on a distal end side of the overtube (64), a side wall opening portion (68) is provided, wherein by bending the distal end of the insertion portion (12) it can be led out through the side wall opening portion. The endoscope auxiliary system known from this document further comprises a positioning member (96), which is an inflatable balloon and is disposed outside the overtube (64), and further comprises a deflecting member (70) disposed within the overtube (64) and configured to adjust the orientation of the endoscope. More specifically, said deflecting member is a fixed type guide member made of a plate-shaped transparent member, the outer peripheral surface thereof being fixed to the inner wall surface of the insertion passage (66) of the overtube (64). The guide member further defines a guide surface (70a) which is inclined in a predetermined inclination angle θ. Furthermore, the endoscope auxiliary system known from this document defines a grip portion (62) detachably or integrally connected with an upper end of the overtube (64) to be manually held by an operator. In view of the forgoing, there exists a need in the related art for an improved ultrathin endoscope auxiliary system, which not only is safe, but easy to use and economic as well, so that improved diagnosis and/or treatment may be delivered to the subject in need.

### SUMMARY

The following presents a simplified summary of the disclosure in order to provide a basic understanding to the reader. This summary is not an extensive overview of the disclosure and it does not identify key/critical elements of the present invention or delineate the scope of the present invention. Its sole purpose is to present some concepts disclosed herein in a simplified form as a prelude to the more detailed description that is presented later.

The present disclosure is directed to an endoscope auxiliary system for assisting the insertion of an ultrathin endoscopic into a subject during DPOCS. The ultrathin endoscope auxiliary system with the features defined in claim 1 comprises an overtube configured to receive the ultrathin endoscope therein; and a mouth piece configured to work with the overtube having the received ultrathin endoscope therein and thereby prevents the ultrathin endoscope from bending or looping in the gastrointestinal tract of the subject during DPOCS. Specifically, the overtube comprises a transparent cap, a side opening, a deflecting member and a positioning member. The transparent cap is disposed at the front end of the overtube; the side opening is disposed on the overtube and approximates to the front end of the overtube; the deflecting member is disposed within the overtube and configured to adjust the orientation of the ultrathin endoscopic; and the positioning member is disposed outside the overtube and approximates to the side opening, and is configured to hold the front end of the overtube in place without sliding in the subject. The deflecting member is capable of deflecting the ultrathin endoscope and thereby allows the ultrathin endoscope to be extended outside the overtube through the side opening. Moreover, the mouth piece is configured to cooperate with the overtube having the received ultrathin endoscope therein to adjust the length and the axial orientation of the overtube and the ultrathin endoscope in the subject.

According to one embodiment of the present disclosure, the positioning member is a first balloon.

According to the present disclosure, the deflecting member comprises an elevating member and an operating member. The elevating member is disposed within the overtube and approximates to the front end of the overtube, wherein the elevating member is configured to support the ultrathin endoscope and thereby allows the ultrathin endoscope to be extended outside the overtube through the side opening. The operating member is coupled to the elevating member and configured to control the elevating member. In one embodiment, the elevating member consists of multiple plates.

According to one embodiment of the present disclosure, the operating member comprises a wire. The wire is coupled to the elevating member and configured to control the elevating member.

In another embodiment of the present disclosure, the deflecting member comprises a second balloon and an inflating member. The second balloon is disposed at the front end of the overtube and opposite to the side opening. The inflating member is coupled to the second balloon and configured to inflate or deflate the second balloon.

According to other embodiments of the present disclosure, the ultrathin endoscope auxiliary system further comprises at least two third balloons which are disposed at the front end and within the overtube. The third balloons are configured to hold the ultrathin endoscope in position so that the ultrathin endoscope cannot slide or rotate.

According to one embodiment of the present disclosure, the overtube further comprises an instrument conduit for receiving a medical instrument there through. The deflecting member is configured to deflate the medical instrument so that it can be extended outside the overtube through the side opening.

An exemplary method of using an ultrathin endoscope in combination with the ultrathin endoscope auxiliary system according to one of the embodiments of the present disclosure to perform cholangioscopy in a subject comprises the following steps of:
(a) fitting the mouth piece into the mouth of the subject;
(b) passing the overtube and the ultrathin endoscope through the mouth piece to allow the overtube, together with the ultrathin endoscope inserted therein, to pass through the esophagus, the stomach, and into the duodenum of the subject;
(c) pressing the positioning member against the duodenum so as to keep the front end of the overtube in place;
(d) adjusting the respective lengths and axial orientations of the overtube and the ultrathin endoscope in the stomach of the subject by pulling or pushing the overtube and the ultrathin scope toward the oral side or the anal side of the subject; and
(e) using the mouth piece to keep the back end of the overtube in place.

According to one example of the present disclosure, the ultrathin endoscope is inserted into the overtube before the step (b).

According to one example of the present disclosure, the transparent cap allows a camera embedded at the front end of the ultrathin endoscope. Moreover, the method may further comprise making a diagnosis based on the captured image.

According to one example of the present disclosure, in the step (c), the positioning member is pressed against the duodenum at a site adjacent to the Ampulla of Vater of the subject.

In one example, the method further comprises the steps of:
(f) activating the deflecting member to support and guide the ultrathin endoscope until it is extended outside the overtube through the side opening; and
(g) pushing the ultrathin endoscope into the bile duct of the subject.

In another example, the method further comprises the step (h) of capturing an image using the camera embedded in the front end of the ultrathin endoscope.

In another example, the method further comprises the steps of:
(i) inserting a medical instrument into the instrument conduit;
(j) activating the deflecting member to support and push the medical instrument until it is extended outside the overtube through the side opening; and
(k) using the medical instrument to provide a treatment to the bile duct based on the captured image in the step (h).

The details of one or more embodiments of this disclosure are set forth in the accompanying description below. Other features and advantages of the invention will be apparent from the detail descriptions, and from the appended claims.

It is to be understood that both the foregoing general description and the following detailed description are by examples, and are intended to provide further explanation of the invention as claimed.

### BRIEF DESCRIPTION OF DRAWINGS

The invention can be more fully understood by reading the detailed description of the invention with reference to the accompanying drawings as follows:
**Figure 1** is a schematic drawing of an ultrathin endoscope auxiliary system 100 in accordance with one embodiment of this invention;
**Figure 2** is a schematic drawing illustrating the overtube 210 according to one embodiment of the present disclosure;
**Figure 3a** is a schematic drawing illustrating functional relationship between an overtube 310 and a mouth piece 350 during DPOCS;
**Figure 3b** is a sectional view of the overtube 310 and the mouth piece 350 of **Figure 3a**;
**Figure 4a-4c** are sectional views of the overtube 410 according to one embodiment of the present disclosure;
**Figure 5a-5b** are sectional views of the overtube 510 according to one embodiment of the present disclosure;
**Figure 6a-6b** are sectional views of the overtube 610 according to one embodiment of the present disclosure;
**Figure 7a** is a schematic drawing illustrating how a medical instrument 790 may perform treatment with the aid of an ultrathin endoscope 750 within the overtube 710 during DPOCS;
**Figure 7b** is a cross-sectional view of the overtube 710, the ultrathin endoscope 750 and the medical instrument 790 of **Figure 7a**;
**Figure 8a-8c** are schematic drawings illustrating the installation of the ultrathin endoscope auxiliary system 100 into an subject; and
**Figure 9** is a schematic drawing illustrating the collaboration of the overtube 710, the ultrathin endoscope auxiliary 760 and the medical instrument 790 during the DOCPS procedure.

### DETAILED DESCRIPTION OF THE INVENTION

The detailed description provided below in connection with the appended drawings is intended as a description of the present disclosure and is not intended to represent the only forms in which the present disclosure may be constructed or utilized.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1 %, or 0.5% of a given value or range. Alternatively, the term "about" means within an acceptable standard error of the mean when considered by one of ordinary skill in the art. Other than in the operating/working examples, or unless otherwise expressly specified, all of the numerical ranges, amounts, values and percentages such as those for quantities of materials, durations of times, temperatures, operating conditions, ratios of amounts, and the likes thereof disclosed herein should be understood as modified in all instances by the term "about." Accordingly, unless indicated to the contrary, the numerical parameters set forth in the present disclosure and attached claims are approximations that can vary as desired. At the very least, each numerical parameter should at least be construed in light of the number of reported significant digits and by applying ordinary rounding techniques.

The term "diagnosis" herein means that a doctor or an attending physician making medical decision(s) such as how to treat the lesion within the bile duct of the subject, based on the images captured by the endoscope of the present disclosure.

The term "treat" and "treatment" are used interchangeably and refer to the use of the ultrathin endoscope auxiliary system of the present invention with the ultrathin endoscope, to sample, wash, clean or perform hemostasis at the lesion site of the bile duct in the subject.

The term "ultrathin endoscope" as used herein indicates an endoscope with a diameter smaller than 6 mm.

As used herein, the term "forward-viewing" means that the images are captured by the camera disposed at the front end of the endoscope. Therefore, the term "forward-viewing type endoscope" means that the endoscope is equipped with the camera which is disposed at the front end of the endoscope. For example, most gastroscopes are forward-viewing type endoscopes.

Here, the term "side-viewing" refers to the images captured by the camera disposed at the lateral side of the endoscope. Therefore, the term "side-viewing type endoscope" refers to the endoscope equipped with the camera which is disposed at the lateral side of the endoscope. For example, most duodenoscopes are side-viewing type endoscopes.

The term "axial orientation" as used herein refers to the longitudinal direction of the ultrathin endoscope or the longitudinal direction of the overtube of the present disclosure.

In the context of the present disclosure, "the front end" of the overtube refers to the end of the overtube that is inserted into the body. By contrast, "the back end" of the overtube means the end of the overtube that is opposite from the front end or the end of the overtube that is close to the mouth piece of the ultrathin endoscope auxiliary system of the present disclosure. Further, throughout this specification, when an overtube of the present disclosure is mentioned in connection with DPOCS procedure, it is intended to mean the overtube having inserted therein an ultrathin endoscope for performing DPOCS, unless specific description is provided to the contrary.

The term "subject" refers to a mammal that is treatable with an ultrathin endoscope and the ultrathin endoscope auxiliary system of the present invention. Said subject includes, but are not limit to, human and non-human primates; such as canine, cat, horse, sheep, swine, cattle etc. The term "subject" is intended to refer to both the male and female gender unless one gender is specifically indicated. Preferably, the subject suitable to be treated by the system or method provided herein is the human species.

One aspect of the present disclosure is to provide an ultrathin endoscope auxiliary system that is novel, economical and easy-to-operate. The ultrathin endoscope auxiliary system is particularly suitable for use with a conventional ultrathin endoscope to perform the DPOCS.

Nowadays, even though using an ultrathin endoscope to implement DPOCS has become a prevalent choice among physicians, yet it is not without limits. For examples, due to its long length and small size (i.e., smaller cross sectional area), the ultrathin endoscope tends to bend and loop within the gastrointestinal tract of the subject, resulting the ultrathin endoscope fails to reach its intended destination, i.e., the bile duct; and hence, unable to complete DPOCS. To address such problem, the inventor of the present application designs a novel auxiliary system for use with an ultrathin endoscope, which may aid in increasing the successful rate of ultrathin endoscopic surgery.

Accordingly, it is the first objective of this invention to provide a novel ultrathin endoscope auxiliary system, which may solve the afore-mentioned looping problems often encounter when the conventional ultrathin endoscope is employed to implement the DPOCS. Further, unlike the DPOCS conducted by use of a mother-baby scope system, in which two attending physicians are required for the task; DPOCS performed under the aid of the ultrathin endoscope auxiliary system of the present invention requires only one physician, and thus will greatly reduce the operating cost.

The ultrathin endoscope auxiliary system of the present invention is designed to work with a conventional ultrathin endoscope during DPOCS, and comprises an overtube and a mouth piece. Moreover, the endoscope auxiliary system of the present invention may facilitate the ultrathin endoscope to capture images in both the forward-viewing mode and the side-viewing mode; thus eliminating the need of a dual endoscope system, for a single endoscope is sufficient to achieve the functions attainable only when a dual endoscope system (e.g., a mother-and-baby-scope system) is employed. Furthermore, during DPOCS, the ultrathin endoscope auxiliary system of the present disclosure may provide the user two supporting positions (or leverage points) for easily adjusting the length and axial orientation of the overtube and the ultrathin endoscope, so as to prevent them from looping in the gastrointestinal tract of the subject. Thus, the ultrathin endoscope auxiliary system of the present disclosure can address the looping problem commonly associated with the use of ultrathin endoscope, and facilitate the insertion of the ultrathin endoscope into the bile duct of the subject.

During DPOCS, the overtube of the present ultrathin endoscope auxiliary system may help positioning the ultrathin endoscope in the subject by taking advantages of the two supporting positions respectively provided by the positioning member and the mouth piece of the present auxiliary system. During DPOCS, the front end of overtube is first held in place (i.e., without sliding or moving) in the subject with the aid of the positioning member. Specifically, the positioning member provides a first supporting position (or leverage point) to the overtube, so that the physician, whom conducts DPOCS, may lean on when adjusting the length and the axial orientation of the overtube (i.e, the overtube having an ultrathin endoscope received therein) in the subject. Upon being adjusted to an acceptable status, the back end of the overtube is then held in place with the aid of the mouth piece, which acts as an additional supporting member. In sum, the overtube is held in place in the subject at the first and second supporting positions described above respectively with the aid of the positioning member and the mouth piece. Thus, the overtube serves as a conduit allowing the ultrathin endoscope to be inserted there through, passing the stomach, esophagus and finally, into the duodenum of the subject. Furthermore, the overtube positioned in the subject in the afore-described manner allows the user sufficient leverage to push or pull the ultrathin endoscope until it reaches a suitable position to be push into and/or pull out of the bile duct of the subject easily.

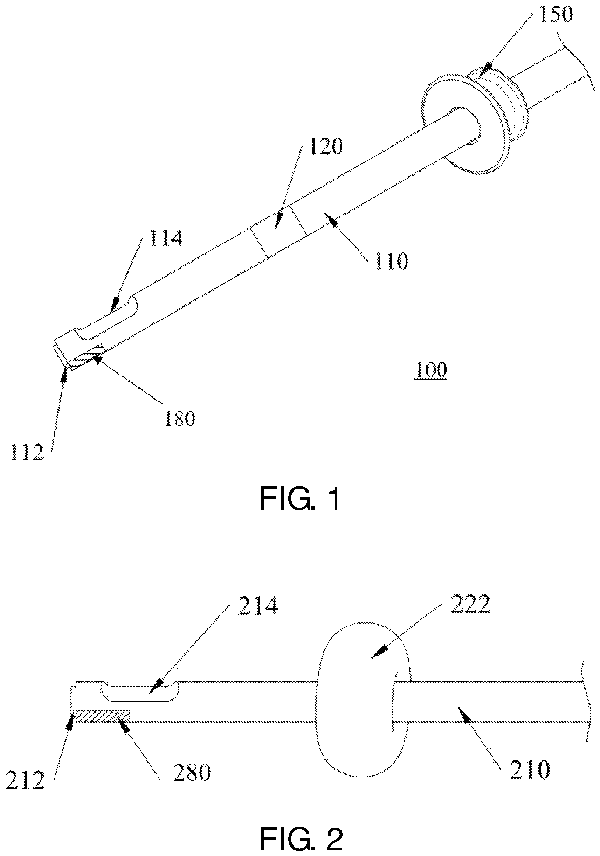

Figure 1 is a schematic drawing of an ultrathin endoscope auxiliary system 100 in accordance with one embodiment of this invention. The ultrathin endoscope auxiliary system 100 comprises an overtube 110, and a mouth piece 150, wherein the overtube 110 is configured to allow the ultrathin endoscope to be inserted therein. Specifically, the overtube 110 comprises a transparent cap 112, a side opening 114, a positioning member 120 and a deflecting member 180. The transparent cap 112 is disposed at the front end of the overtube, so that the ultrathin endoscope inserted in the orvertube 110 may capture the images in the forward-viewing mode through the transparent cap 112 of the overtube110. Further, the ultrathin endoscope can be held against the transparent cap 112 to allow the overtube 110 to be pushed into the subject. In the context of the present disclosure, the "front end of the overtube" refers to the end of the overtube to be inserted into the subject unless the otherwise specified.

The side opening 114 is disposed on and approximates to the front end of the overtube 110. The deflecting member 180 is disposed within and approximates to the front end of the overtube 100, and is configured to adjust the orientation of the ultrathin endoscope (not shown) therein by increasing the bending angle of the ultrathin endoscope, and thereby allows the ultrathin endoscope to be extended outside the overtube 110 through the side opening 114. The positioning member 120 is disposed outside the overtube 110 and approximates to the side opening 114. The positioning member 120 is configured to hold the front end of the overtube 110 in place so that it does not move or slide in the subject. According to various embodiments of the present disclosure, the positioning member 120 may be a balloon. In some examples, instead of being filled with air, the balloon may be filled with a solution containing a contrast agent. The mouth piece 150 is configured to allow the overtube 110 with or without the ultrathin endoscope being inserted therein to pass through and to hold the overtube 110 in place; it may also collaborate with the overtube 110 during DPOCS when the user tries to adjust the length and the axial orientation of the overtube 110 having an ultrathin endoscope therein in the subject.

In one example, the diameter of the overtube 110 is about 8-16 mm; such as 8, 9, 10, 11, 12, 13, 14, 15 or 16 mm. Preferably, the diameter is about 10-14mm, such as 10, 11, 12, 13, or 14 mm. Most preferably, the diameter is about 11-13 mm, such as 11, 12, or 13 mm.

Figure 2 is a schematic drawing illustrating the overtube 210 according to one embodiment of the present disclosure. In this embodiment, the positioning member is a first balloon 222. Specifically, the overtube 210 comprises a transparent cap 212, a side opening 214, a first balloon 222 and a deflecting member 280. The arrangement of the transparent cap 212, the side opening 214 and the deflecting member 280 is similar to that described above in connection with Figure 1 ; for the purpose of brevity, the details of these elements are not described herein.

It should be noted that the first balloon 222 is disposed outside the overtube 210, and approximates to the side opening 214. During DPOCS, the front end of the overtube 210 is held and positioned in the subject by the first balloon 222. In another example, instead of being filled with air, the first balloon 222 may be a filled with a solution containing a contrast agent.

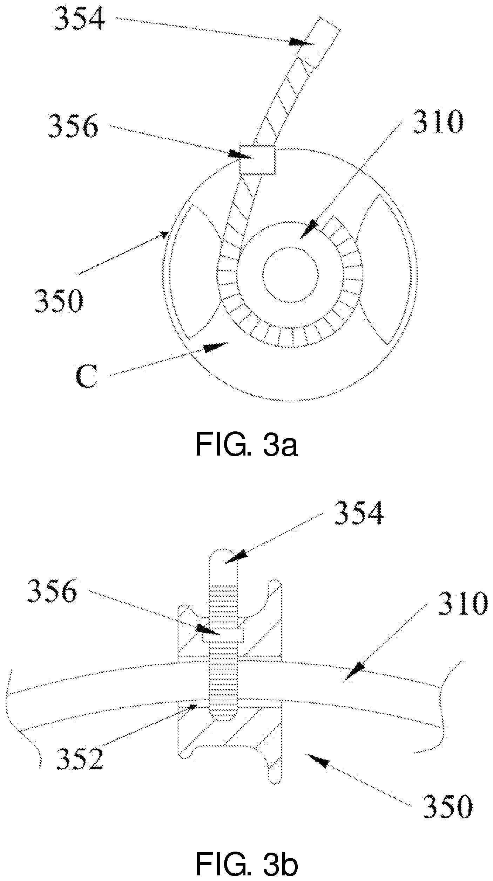

The functional relationship between an overtube 310 and a mouth piece 350 during DPOCS is schematically illustrated in Figure 3a; whereas Figure 3b is the sectional view of the overtube 310 and the mouth piece 350 of Figure 3a. Referring to both Figure 3a and Figure 3b, the mouth piece 350 comprises a passage 352 configured to allow the overtube 310 to pass there through; a strip 354 and a fixing hole 356, which work in a cooperative manner to secure the overtube 310 and thereby preventing it from dislodging or moving. Specifically, the fixing hole 356 is disposed on the wall of the mouth piece 350 and configures to receive the strip 354 by allowing the strip 354 to pass therethrough and hold it in place to create a tightening force, which in turn may hold the overtube 310 passing through the passage 352 of the mouth piece 350 in place. The strip 354 is disposed within and along the inner surface of the passage 352 and forms a circle (C). While in practice, one end of the strip 354 is passed through the fixing hole 356 and the size of the circle (C) (or the diameter of C) may be changed depending on how tight the user wishes to hold the overtube 310 in place by pulling and adjusting the length of the strips 354 that passes through the fixing hole 356. In other words, if a tighter position of the overtube 310 is sought, the strip 354 is pulled harder to allow more of it to pass through the fixing hole 356; if not, the strip 354 is released by allowing less of it to pass through the fixing hole 356. Further, the strip 354 is removable, which means it is placed onto the mouth piece 350 to help securing the overtube 310 only when it is needed.

Further note that the strip 354 disclosed in Figure 3 is merely an example of the fastening member of the present invention. In other words, in other examples of the disclosure, the mouth piece 350 may be used with fastening member(s) other than the strip 354 exemplified in this example. Further, a screw, may be used in lieu of the fixing hole 356 exemplified in this example. The screw may be oriented to be vertical to the longitudinal orientation (axial) of the passage 352, for the purpose of holding the strip 354 in place and thereby securing the overtube 310.

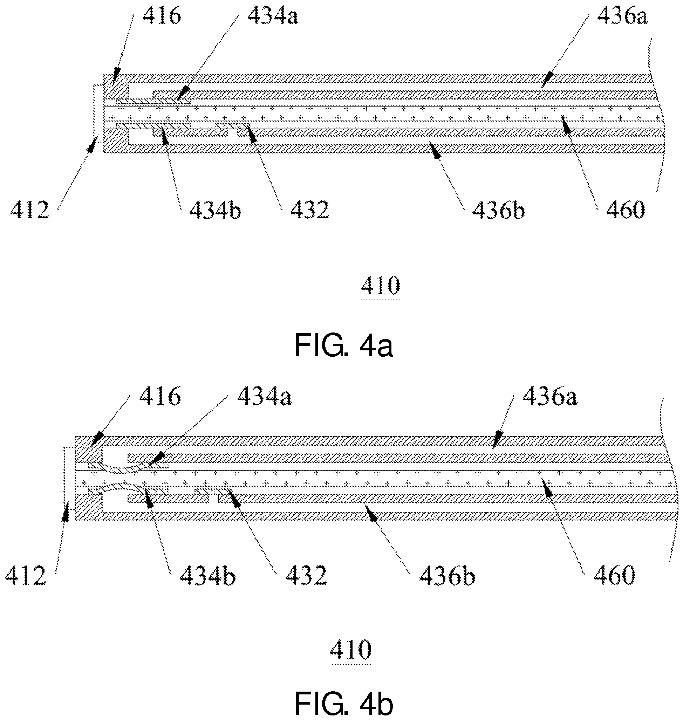

Figures 4a to 4c are sectional views of an overtube 410 according to another embodiment of the present disclosure. The overtube 410 of this embodiment is structurally similar to the overtube 110 of Figure1, except additional two balloons (or the third balloons (434a, 434b)) are disposed within and at the front end of the overtube 410, for holding the ultrathin endoscope 460 in place. Moreover, the deflecting member comprises an elevating member and an operating member. The elevating member is also a balloon, i.e., the second balloon 432. The operating member may be a machine, designed to supply air to the second balloon 432.

In this example, a transparent cap 412 is disposed at the front end of the overtube 410. The third balloons (434a, 434b) are disposed on the inner wall 416 of the overtube 410, opposite to each other, and approximate to the transparent cap 412, for holding the ultrathin endoscope 460 in place. The second balloon 432 is also disposed on the inner wall 416 of the overtube 410, next to the third balloons (434a, 434b) and opposite to the side opening 414, for adjusting the axial orientation of the overtube 460.

Referring to Figures 4a to 4c, the overtube 410 further comprises two air conduits (436a, 436b). The air conduits (436a, 436b) are configured to couple with the first balloon (not shown), the second balloon 432, and the third balloons (434a, 434b) for the purpose of inflating or deflating each balloon. Specifically, one end of the air conduits (436a, 436b) is coupled to the machine (e.g., a pump, not shown) designed to supply air to the second balloon 432 and the third balloons (434a, 434b). Furthermore, the balloon may be inflated or deflated to various sizes according to the actual needs. In certain examples, the air conduits (436a, 436b) may be connected to the machine (not shown) via a catheter (not shown).

During DPOCS, the second balloon 432 and the third balloons (434a, 434b) are initially flat, or without being filled with air. Once the ultrathin endoscope 460 is disposed between the third balloons (434a, 434b) and is on or above the second balloon 432, the machine designed to supply air (e.g., a pump) (not shown) may then be activated to provide air to expand the third balloons (434a, 434b) until they respectively reach a size sufficient enough to hold the front end of the ultrathin endoscope 460 tightly in place, so that the ultrathin endoscope 460 within the overtube 410 cannot move or slide, or slipped out from the overtube 410.. Further, during DPOCS, due to the transparent cap 412 disposed in the front end of the overtube 410, it allows the ultrathin endoscope 460 to capture *in vivo* images in the forward-viewing mode.

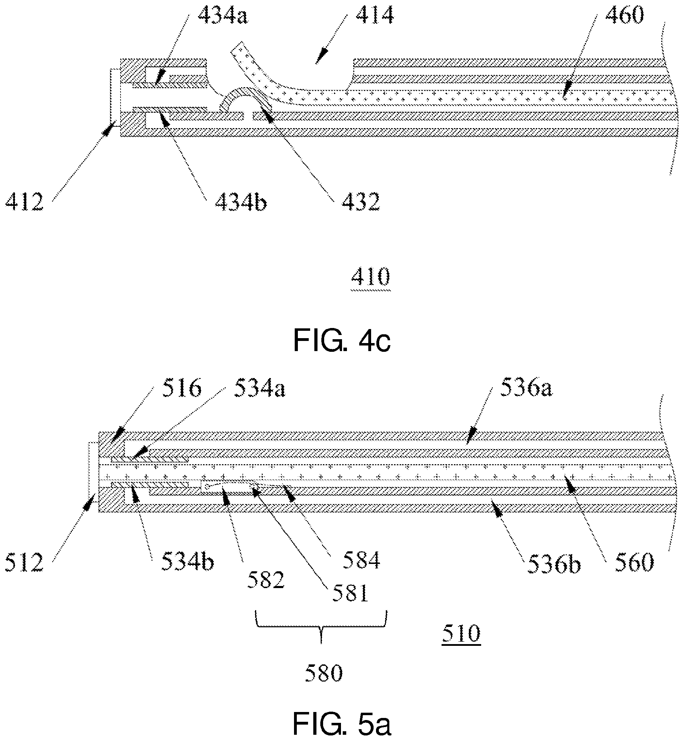

Referring again to Figures 4a and 4c, the orientation of the ultrathin endoscope 460 may be adjusted via controlling the size or the volume of the second balloon 432 (deflating or inflating). The second balloon 432 is initially flat (see Figure 4a), and only when the ultrathin endoscope 460 is pushed and reached the position right above the second balloon 432, and has captured images in the forward-viewing mode through the transparent cap 412 of the overtube 410; then the machine designed to supply air (not shown) is activated to inflate the second balloon 432 (see Figure 4c), the inflated second balloon 432 may then lend support to, and guide the ultrathin endoscope 460, by pushing the ultrathin endoscope 460 in an upward motion (i.e., by increasing the upward angle of the ultrathin endoscope 460) until it eventually extends out of the overtube 410 through the side opening 414; in which state, the ultrathin endoscope 460 may then capture images in the forward-viewing mode. Therefore, the ultrathin endoscope 460 may work with the overtube 410 of the present auxiliary system and achieve the functions independently provided by the forward view endoscope and the side view endoscope of this art.

Further, the second balloon 432 may act as a barricade during DPOCS. In this case, when the ultrathin endoscope 460 is pushed toward to the front end of the overtube 410, the inflated second balloon 432 may lend support to the ultrathin endoscope 460, by allowing the ultrathin endoscope 460 to be pushed against the inflated second balloon 432 and eventually slides out of the overtube 410 through the side opening 414 and into the bile duct of the subject.

According to various examples of the present disclosure, the first balloon (not shown), the second balloon 432 and the third balloons (434a, 434b) are respectively made from resin (e.g., silicon or latex) or biocompatible materials.

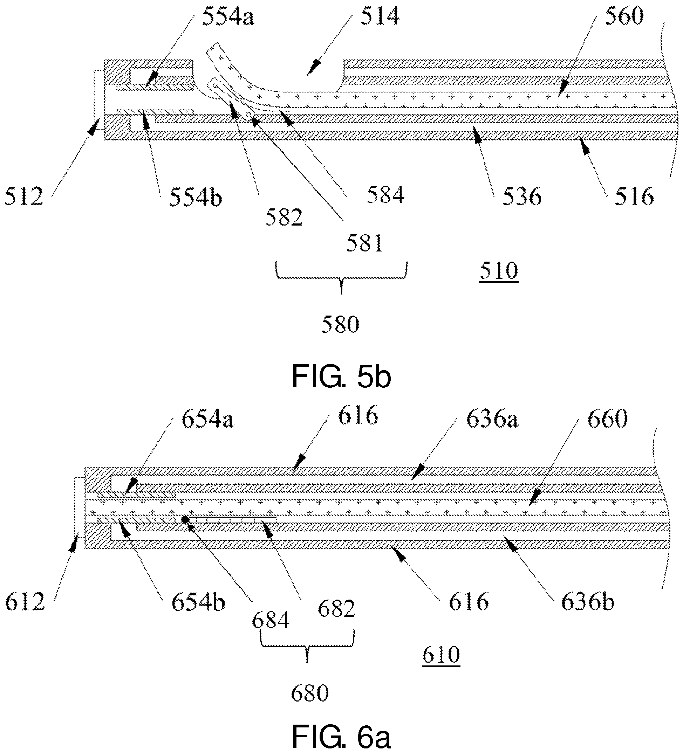

Figures 5a and 5b are sectional views of the overtube 510 according to another example of the present invention, in which an alternative example of the deflecting member is disclosed. In this example, the deflecting member 580 comprises an elevating member and an operating member, wherein the elevating member consists of a pin 581 and a slat 582. The operating member comprises a wire 584. The rest of the elements and their arrangement of the overtube 510 remain relatively the same as those depicted in Figure 4.

Similarly, the overtube 510 of this example also comprises: a transparent cap 512, two of the third balloons (534a, 534b), two air conduits (536a, 536b), the pin 581, the slat 582 and the wire 584, wherein the transparent cap 512, the third balloons (534a, 534b) and the air conduits (536a, 536b) are arranged in the same manner as those depicted in Figure 4; therefore, detail description of these same elements are omitted herein for the purpose of brevity,.

In this example, the deflecting member 580 is configured to support and guide the ultrathin endoscope 560 to a different orientation. The slat 582 is disposed on the inner wall 516 of the overtube 510, next to the third balloon 534b, and opposite to the side opening 514 (see Figure 5b); and one side of the slat 582 is fixed onto the sidewall of the overtube 510 by the pin 581, whereas the other side of the slat 582 is connected to the wire 584. During DPOCS, when the ultrathin endoscope 560 needs to be directed to another direction, such as to be extended out of the overtube 510, the user may pull the wire 584 so that the slat 582 is pulled upward and thereby supporting the ultrathin endoscope 560 and directing it outward through the side opening 514 of the overtube 510 and into the bile duct of the subject.

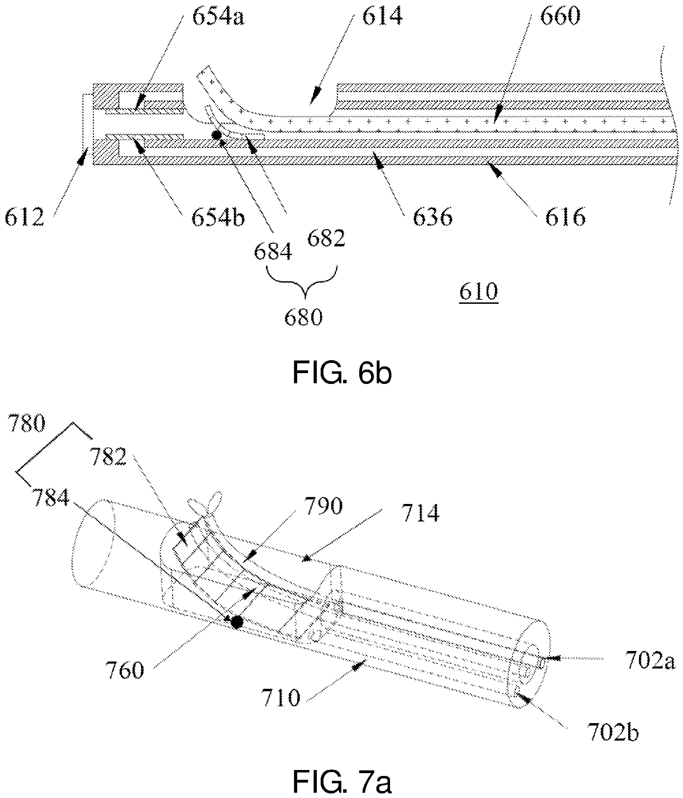

Another example of the deflecting member is illustrated in Figures 6a and 6b. In this example, the overtube 610 is constructed to comprise a transparent cap 612, two of the third balloons (634a, 634b), two air conduits (636a, 636b), and a deflecting member 680, in which the deflecting member 680 consists of an elevating member and an operating member. The elevating member is comprised of a plurality of slats 682. The operating member comprises a mini motor 684, electrically coupled to the plurality of slats 682. Further, the transparent cap 612, the third balloons (634a, 634b) and the air conduits (636a, 636b) are arranged in the same manner as those depicted in Figure 4; therefore, detail description to these elements is omitted herein for the purpose of brevity.

During DPOCS, when the ultrathin endoscope 660 needs to be directed to another direction, such as to be extended out of the overtube 610 and into the bile duct of the subject, the mini motor 684 is activated to drive the plurality of slats 682 upward and thereby act as a support to the ultrathin endoscope 660 and further directs it outward through the side opening 614 of the overtube 610 and into the bile duct of the subject. In extreme condition, the plurality of slats 682 may be driven by the mini motor 684 to a nearly perpendicular position relative to the longitudinal direction of the overtube 610. In an alternative example, the plurality of slats 682 may be driven or controlled by a wire instead of the mini motor 684 (see Figure 6b).

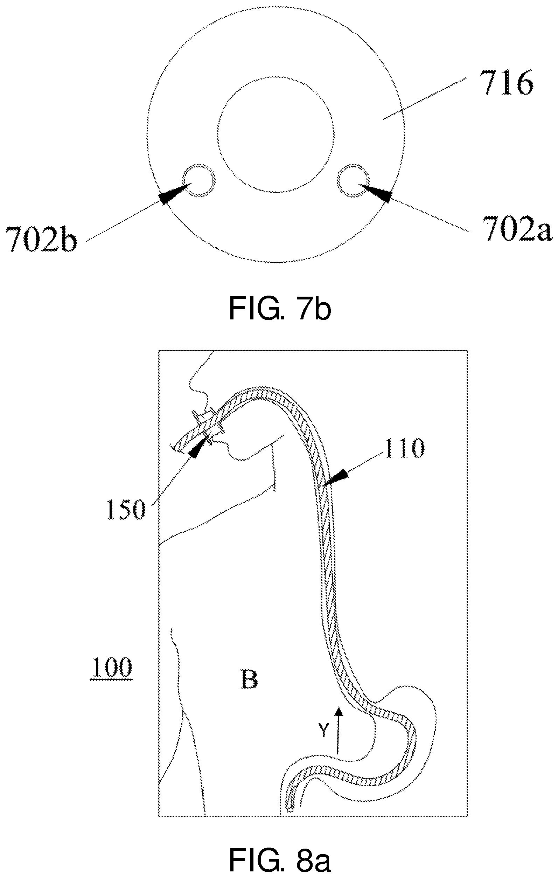

Figure 7a is a schematic drawing illustrating how a medical instrument 790 may perform treatment with the aid of an ultrathin endoscope 760 and the overtube710 of the present disclosure during DPOCS according to one embodiment of the present disclosure. In this embodiment, the overtube 710 further comprises at least one instrument conduits for receiving medical tools or instruments there through. Once the attending physician has captured imagines and made diagnosis to the lesion with the aid of an ultrathin endoscope and the auxiliary system of the present disclosure, medical treatment (e.g., hemostasis, surgery, and/or sampling, washing, cleaning of the lesion) may be provided to the lesion by sending proper tools through the at least one instrument conduits. Figure 7b is a cross-sectional view of the overtube 710 of Figure 7a, in which two instrument conduits (702a, 702b) are illustrated.

Referring to Figure 7a and Figure 7b, the overtube 710 comprises two instrument conduits (702a, 702b), respectively disposed within the overtube 710 along the longitudinal direction of the overtube 710 and are paralleled to each other. As depicted in Figure 7a, both the ultrathin endoscope 760 and the medical instrument 790 are disposed above the deflecting member 780, which in this example, is consisted of a plurality of slats 782 and a mini motor 784. Again, the mini motor 784 is driven to push the plurality of slats 782 upward by bending them in an upward direction until they reach an acceptable position that may support and allow both the ultrathin endoscope 760 and the medical instrument 790 to be extended out of the overtube 710 through the side opening 714, and into the bile duct. Then, with the aid of both the ultrathin endoscope 760 and the medical instrument 790, the physician may provide treatment to the lesion. The medical instrument 790 includes, but is not limited to, an argon plasma coagulation catheter, forceps bending cannula, a basket, or any other pediatric medical tools.

Another aspect of this invention is directed to a method of using the ultrathin endoscope auxiliary system on a subject when cholangioscopy, especially DPOCS, is performed.

As indicated in the "background" section, DPOCS conducted using single endoscope is deeply troubled by its inherent looping problem, resulting physicians revert to the more expensive dual endoscope system, in which two physician are required for this procedure. Inventors of the present invention proposed a novel ultrathin endoscope auxiliary system, which may be used corporately with the conventional ultrathin endoscope to implement DPOCS, thus eliminate the need of a more expensive dual endoscope system, and since only one physician is needed to perform DPOCS, the labor cost is greatly reduced. Further, the ultrathin endoscope auxiliary system of the present disclosure also eliminates the inherent looping problem associated with ultrathin endoscope during DPOCS, thus making DPOCS possible using just one ultrathin endoscope.

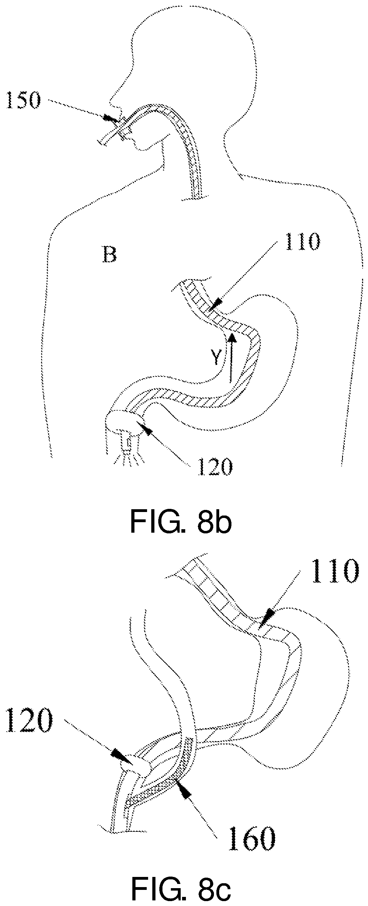

Referring to Figure 1 and Figures 8a-8c, DPOCS begins by fitting a mouth piece 150 of the ultrathin endoscope auxiliary system 100 into the mouth of a subject (B). Tthen, one end of the overtube 110 (i.e., the front end) is passed through the mouth piece 150 and secured thereto by use of a strip (not shown). An ultrathin endoscope (not shown) is then inserted through the mouth piece 150 and into the overtube 110, and together, the overtube 110 and the ultrathin endoscope are pushed into the stomach of the subject (B). At this time, the ultrathin endoscope inserted in the overtube 110 may capture the images in the forward-viewing mode through the transparent cap 112 of the overtube 110. The overtube 110 along with the inserted ultrathin endoscope are continuously being pushed deeper into the subject, passing the stomach and finally into the duodenum of the subject (B) (see Figure 8a).

Once reached the duodenum, the positioning member 120 is pressed against the duodenum so as to prevent the front end of the overtube 110 from moving or sliding out of its current position. The positioning member 120, at this position, provides a first leverage point or supporting force to keep the front end of the overtube 110 in place in the subject.
Then, the overtube 110 along with the ultrathin endoscope inserted therethrough in the stomach are pulled toward the oral site of the subject (B), so as to reduce the respective lengths of the overtube 110 and the ultrathin endoscope in the stomach, as well as to adjust the axial orientation of the overtube 110 and the ultrathin endoscope in the subject (B) until they are nearly parallel to the height orientation (or Y-axis) of the subject (B). Alternatively, the overtube 110 and the ultrathin endoscope inserted there through may be pushed further into the subject toward the anal side of the subject (B) for the same purpose of adjusting the respective lengths and the axial orientations of the overtube 110 and the ultrathin endoscope in the subject. Once the respective lengths and axial orientation of the overtube 110 and the ultrathin endoscope have been properly adjusted, the back end of the overtube 110 (that is, the end opposite to the front end of the overtube 110) is held in place with the aid of the mouth piece 150.

In the afore-mentioned step, in which the respective lengths and axial orientation of the overtube and the ultrathin endoscope are adjusted within the subject, particularly within the stomach of the subject, it cannot be accomplished without the position member and the mouth piece of the present ultrathin endoscope auxiliary system. Specifically, the position member and the mouth piece respectively act as supporting elements at their respective positions within the subject (i.e., duodenum and mouth), by providing levering points to the push and/or pull forces for adjusting the length and axial orientation of the overtube, as well as the ultrathin endoscope; which in turn, eliminates the inherent looping problem commonly associated with ultrathin endoscope in DPOCS.

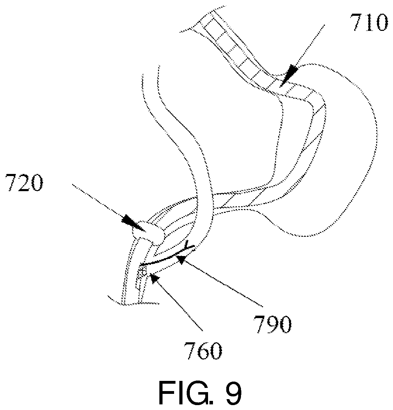

Once the length and the axial orientation of the overtube 110 and the ultrathin endoscope have been adjusted, the deflecting member 180 of the overtube 110 is activated by bending upward to an extent to guide and support the ultrathin endoscope 160 until the ultrathin endoscope 160 may be extended outside the overtube 110 through the side opening 114 (see Figure 8c). The front end of the ultrathin endoscope 160, once outside of the overtube 110, may capture images in forward-viewing mode by the embedded camera thereon; such function generally can only be accomplished with an endoscope equipped with a side-viewing camera. Further, once the front end of the ultrathin endoscope 160 has been pushed out of the overtube 110 through the side opening 114, it may then be pushed further into the bile duct of the subject (B) for subsequent medical treatment(s). In this regard, the present ultrathin endoscope auxiliary system may further comprise at least one instrument conduits, in which suitable medical instrument(s) may be delivered to the lesion site and provide treatments thereto. The ordinary skill in the art should understand that the number and the types of the medical instruments used herein may vary on the clinical situation. For example, a physician may use the present ultrathin endoscope auxiliary system, together with at least two medical instruments and one ultrathin endoscope, for the diagnosis and/or treatment of the bile duct related disease. As depicted in the exemplified Figure 9, a medical instrument 790 is pushed out of the overtube 710 through its side opening 714and into the bile duct to perform a required treatment.

It will be understood that the above description of embodiments is given by way of example only and that various modifications may be made by those with ordinary skill in the art. The above specification, examples and data provide a complete description of the structure and use of exemplary embodiments of the invention.

## Claims

1. An ultrathin endoscope auxiliary system (100) for assisting the insertion of an ultrathin endoscope (160, 460, 560, 660, 760) into a subject, comprising:
an overtube (110, 210, 310, 410, 510, 610, 710), configured to allow the ultrathin endoscope (160, 460, 560, 660, 760) to be inserted therein, wherein the overtube (110, 210, 310, 410, 510, 610, 710) comprises:
a transparent cap (112, 212, 412, 512, 612), disposed at the front end of the overtube (110, 210, 310, 410, 510, 610, 710);
a side opening (114, 214, 414, 514, 614, 714), disposed on the overtube (110, 210, 310, 410, 510, 610, 710) and approximating to the front end of the overtube (110, 210, 310, 410, 510, 610, 710);
a deflecting member (180, 280, 580, 680, 780), disposed within the overtube (110, 210, 310, 410, 510, 610, 710) and configured to adjust the orientation of the ultrathin endoscope (160, 460, 560, 660, 760), wherein the deflecting member (180, 280, 580, 680, 780) comprises:
an elevating member, disposed within the overtube (110, 210, 310, 410, 510, 610, 710) and approximating to the front end of the overtube (110, 210, 310, 410, 510, 610, 710), configured to deflect the ultrathin endoscope (160, 460, 560, 660, 760) in an operation state and thereby allowing the ultrathin endoscope (160, 460, 560, 660, 760) to be extended outside the overtube (110, 210, 310, 410, 510, 610, 710) through the side opening (114, 214, 414, 514, 614, 714); and
an operating member, coupled to the elevating member and configured to control an operation of the elevating member;
a positioning member (120), disposed outside the overtube (110, 210, 310, 410, 510, 610, 710) and approximating to the side opening (114, 214, 414, 514, 614, 714), configured to position the front end of the overtube (110, 210, 310, 410, 510, 610, 710) in place in the subject; and
a mouth piece (150, 350), comprising:a passage (352) configured to allow the overtube (110, 210, 310, 410, 510, 610, 710) to pass there through;
a strip (354); and
a fixing hole (356) configured to allow the strip (354) to pass therethrough, whereby the strip (354) and the fixing hole (356) are configured to work in a cooperative manner to secure the overtube (110, 210, 310, 410, 510, 610, 710) preventing it from moving.

2. The ultrathin endoscope auxiliary system (100) of claim 1, wherein the positioning member (120) is a first balloon (222).

3. The ultrathin endoscope auxiliary system (100) of claim 1, wherein the elevating member consists of multiple slats (682).

4. The ultrathin endoscope auxiliary system (100) of claim 1, wherein the operating member comprises a wire (584) coupled to the elevating member and configured to control the elevating member.

5. The ultrathin endoscope auxiliary system (100) of claim 1, wherein the elevating member consists of
a second balloon (432), disposed at the front end of the overtube (110, 210, 310, 410, 510, 610, 710) and opposite to the side opening (114, 14, 414, 514, 614, 714); and
wherein the operating member comprises an inflating member, coupled to the second balloon (432) and configured to inflate or deflate the second balloon (432).

6. The ultrathin endoscope auxiliary system (100) of claim 1, wherein the overtube (110, 210, 310, 410, 510, 610, 710) comprises at least one instrument conduit (702a, 702b) for receiving a medical instrument (790) there through; and the deflecting member (180, 280, 580, 680, 780) is configured to deflect the medical instrument (790) so that it can be extended outside the overtube (110, 210, 310, 410, 510, 610, 710) through the side opening (114, 214, 414, 514, 614, 714).

7. The ultrathin endoscope auxiliary system (100) of claim 1, further comprising at least two third balloons (434a, 434b, 534a, 534b, 634a, 634b), disposed at the front end of the overtube (110, 210, 310, 410, 510, 610, 710), and configured to hold the ultrathin endoscope (160, 460, 560, 660, 760) in place.

## Patentansprüche

1. Ultradünnes Endoskophilfssystem (100) zur Unterstützung des Einführens eines ultradünnen Endoskops (160, 460, 560, 660, 760) in ein Subjekt, umfassend:
ein Überrohr (110, 210, 310, 410, 510, 610, 710), das darauf ausgelegt ist, dem ultradünnen Endoskop (160, 460, 560, 660, 760) zu ermöglichen, darin eingeführt zu werden, wobei das Überrohr (110, 210, 310, 410, 510, 610, 710) umfasst: eine transparente Kappe (112, 212, 412, 512, 612), die an dem vorderen Ende des Überrohrs (110, 210, 310, 410, 510, 610, 710) angeordnet ist,
eine Seitenöffnung (114, 214, 414, 514, 614, 714), die an dem Überrohr (110, 210, 310, 410, 510, 610, 710) angeordnet ist, und sich dem vorderen Ende des Überrohrs (110, 210, 310, 410, 510, 610, 710) annähert,
ein Ablenkelement (180, 280, 580, 680, 780), das innerhalb des Überrohrs (110, 210, 310, 410, 510, 610, 710) angeordnet ist, und darauf ausgelegt ist, die Orientierung des ultradünnen Endoskops (160, 460, 560, 660, 760) anzupassen, wobei das Ablenkelement (180, 280, 580, 680, 780) umfasst:
ein Hebeelement, das innerhalb des Überrohrs (110, 210, 310, 410, 510, 610, 710) angeordnet ist, und sich dem vorderen Ende des Überrohrs (110, 210, 310, 410, 510, 610, 710) annähert, das darauf ausgelegt ist, das ultradünne Endoskop (160, 460, 560, 660, 760) in einem Betriebszustand abzulenken und dabei dem ultradünnen Endoskop (160, 460, 560, 660, 760) zu erlauben, außerhalb des Überrohrs (110, 210, 310, 410, 510, 610, 710) durch die Seitenöffnung (114, 214, 414, 514, 614, 714) verlängert zu werden, und
ein Betriebselement, das an das Hebeelement gekoppelt ist und darauf ausgelegt ist, einen Betrieb des Hebeelements zu steuern,
ein Positionierelement (120), das außerhalb des Überrohrs (110, 210, 310, 410, 510, 610, 710) angeordnet ist und sich der Seitenöffnung (114, 214, 414, 514, 614, 714) annähert und das darauf ausgelegt ist, das vordere Ende des Überrohrs (110, 210, 310, 410, 510, 610, 710) an einer Position in dem Subjekt zu positionieren, und
ein Mundstück (150, 350), das eine Passage (352) umfasst, die darauf ausgelegt ist, es dem Überrohr (110, 210, 310, 410, 510, 610, 710) zu ermöglichen, dadurch zu verlaufen,
einen Streifen (354) und
ein Befestigungsloch (356), das darauf ausgelegt ist, es dem Streifen (354) zu ermöglichen, dadurch zu verlaufen, wobei der Streifen (354) und das Befestigungsloch (356) darauf ausgelegt sind, in einer kooperativen Weise zu arbeiten, um das Überrohr (110, 210, 310, 410, 510, 610, 710) zu sichern, wobei verhindert wird, dass es sich bewegt.

2. Ultradünnes Endoskophilfssystem (100) gemäß Anspruch 1, bei welchem das Positionierelement (120) ein erster Ballon (222) ist.

3. Ultradünnes Endoskophilfssystem (100) gemäß Anspruch 1, bei welchem das Hebeelement aus mehreren Lamellen (682) besteht.

4. Ultradünnes Endoskophilfssystem (100) gemäß Anspruch 1, bei welchem das Betriebselement einen Draht (584) umfasst, der an das Hebeelement gekoppelt ist und darauf ausgelegt ist, das Hebeelement zu steuern.

5. Ultradünnes Endoskophilfssystem (100) gemäß Anspruch 1, bei welchem das Hebeelement aus
einem zweiten Ballon (432) besteht, der an dem vorderen Ende des Überrohrs (110, 210, 310, 410, 510, 610, 710) angeordnet und entgegengesetzt zu der Seitenöffnung (114, 214, 414, 514, 614, 714) ist, und
wobei das Betriebselement ein Aufpumpelement umfasst, das an den zweiten Ballon (432) gekoppelt ist und darauf ausgelegt ist, den zweiten Ballon (432) aufzupumpen oder zu entleeren.

6. Ultradünnes Endoskophilfssystem (100) gemäß Anspruch 1, bei welchem das Überrohr (110, 210, 310, 410, 510, 610, 710) zumindest eine Instrumentenleitung (702a, 702b) umfasst, um ein medizinisches Instrument (790) dadurch aufzunehmen, und das Ablenkelement (180, 280, 580, 680, 780) darauf ausgelegt ist, das medizinische Instrument (790) abzulenken, so dass es außerhalb des Überrohrs (110, 210, 310, 410, 510, 610, 710) durch die Seitenöffnung (114, 214, 414, 514, 614, 714) verlängert werden kann.

7. Ultradünnes Endoskophilfssystem (100) gemäß Anspruch 1, das ferner zumindest zwei dritte Ballons (434a, 434b, 534a, 534b, 634a, 634b) umfasst, die an dem vorderen Ende des Überrohrs (110, 210, 310, 410, 510, 610, 710) angeordnet sind, und darauf ausgelegt sind, das ultradünne Endoskop (160, 460, 560, 660, 760) in Position zu halten.

## Revendications

1. Système auxiliaire d'endoscope ultra-mince (100) pour aider à l'insertion d'un endoscope ultra-mince (160, 460, 560, 660, 760) dans un sujet, comprenant:
un sur-tube (110, 210, 310, 410, 510, 610, 710), configuré pour permettre à l'endoscope ultra-mince (160, 460, 560, 660, 760) d'être inséré en son sein, dans lequel le sur-tube (110, 210, 310, 410, 510, 610, 710) comprend:
un capuchon transparent (112, 212, 412, 512, 612), disposé à l'extrémité avant du sur-tube (110, 210, 310, 410, 510, 610, 710);
une ouverture latérale (114, 214, 414, 514, 614, 714), disposée sur le sur-tube (110, 210, 310, 410, 510, 610, 710) et se rapprochant de l'extrémité avant du sur-tube (110, 210, 310, 410, 510, 610, 710);
un élément de déviation (180, 280, 580, 680, 780), disposé à l'intérieur du sur-tube (110, 210, 310, 410, 510, 610, 710) et configuré pour ajuster l'orientation de l'endoscope ultra-mince (160, 460, 560, 660, 760), dans lequel l'élément de déviation (180, 280, 580, 680, 780) comprend:
un élément élévateur, disposé à l'intérieur du sur-tube (110, 210, 310, 410, 510, 610, 710) et se rapprochant de l'extrémité avant du sur-tube (110, 210, 310, 410, 510, 610, 710), configuré pour dévier l'endoscope ultra-mince (160, 460, 560, 660, 760) dans un état de fonctionnement et permettre ainsi à l'endoscope ultra-mince (160, 460, 560, 660, 760) d'être étendu à l'extérieur du sur-tube (110, 210, 310, 410 , 510, 610, 710) à travers l'ouverture latérale (114, 214, 414, 514, 614, 714); et
un élément d'actionnement, couplé à l'élément élévateur et configuré pour commander un fonctionnement de l'élément élévateur;
un élément de positionnement (120), disposé à l'extérieur du sur-tube (110, 210, 310, 410, 510, 610, 710) et se rapprochant de l'ouverture latérale (114, 214, 414, 514, 614, 714), configuré pour positionner l'extrémité avant du sur-tube (110, 210, 310, 410, 510, 610, 710) en place dans le sujet; et
un embout buccal (150, 350), comprenant: un passage (352) configuré pour permettre au sur-tube (110, 210, 310, 410, 510, 610, 710) de passer à travers celui-ci;
une bande (354); et
un trou de fixation (356) configuré pour permettre à la bande (354) de passer à travers celui-ci, selon lequel la bande (354) et le trou de fixation (356) sont configurés pour travailler de manière coopérative pour fixer le sur-tube (110, 210, 310, 410, 510, 610, 710) en l'empêchant de bouger.

2. Système auxiliaire d'endoscope ultra-mince (100) selon la revendication 1, dans lequel l'élément de positionnement (120) est un premier ballonnet (222).

3. Système auxiliaire d'endoscope ultra-mince (100) selon la revendication 1, dans lequel l'élément élévateur est constitué de plusieurs lamelles (682).

4. Système auxiliaire d'endoscope ultra-mince (100) selon la revendication 1, dans lequel l'élément d'actionnement comprend un fil (584) couplé à l'élément élévateur et configuré pour commander l'élément élévateur.

5. Système auxiliaire d'endoscope ultra-mince (100) selon la revendication 1, dans lequel l'élément élévateur consiste en
un deuxième ballonnet (432), disposé à l'extrémité avant du sur-tube (110, 210, 310, 410, 510, 610, 710) et à l'opposé de l'ouverture latérale (114, 214, 414, 514, 614, 714); et
dans lequel l'élément d'actionnement comprend un élément de gonflage, couplé au deuxième ballonnet (432) et configuré pour gonfler ou dégonfler le deuxième ballonnet (432).

6. Système auxiliaire d'endoscope ultra-mince (100) selon la revendication 1, dans lequel le sur-tube (110, 210, 310, 410, 510, 510, 710) comprend au moins un conduit d'instrument (702a, 702b) pour recevoir un instrument médical (790) à travers celui-ci ; et l'élément de déviation (180, 280, 580, 680, 780) est configuré pour dévier l'instrument médical (790) de sorte qu'il puisse être étendu à l'extérieur du sur-tube (110, 210, 310, 410, 510, 610, 710) à travers l'ouverture latérale (114, 214, 414, 514, 614, 714).

7. Système auxiliaire d'endoscope ultra-mince (100) selon la revendication 1, comprenant en outre au moins deux troisièmes ballonnets (434a, 434b, 534a, 534b, 634a, 634b), disposés à l'extrémité avant du sur-tube (110, 210, 310, 410, 510, 610, 710) et configurés pour maintenir l'endoscope ultra-mince (160, 460, 560, 660, 760) en place.
